# EUROPEAN PATENT APPLICATION

(11) **EP 3 075 418 A1**
(43) Date of publication of application: **05.10.2016**
(21) Application number: 15164235.2
(22) Date of filing: 20.04.2015
(51) Int. Cl.: A61Q 19/00, A61K 8/42, A61K 8/87

(54) **UREA BASED SKIN TREATMENT**

(30) Priority: 01.04.2015 NZ 70663115
(71) Applicant: Neat Feat Products Limited, Auckland (NZ)
(72) Inventor: Roberts, David, Brisbane, Queensland (AU)
(74) Representative: Sayer, Robert David

(57) **Abstract**

A topical formulation for treating human skin, having:
• urea;
• film forming polymer;
• humectant;
• preservative; and
• water

the above ingredients being combined such that the preservative protects the formulation against bacterial invasion, the humectant assists the formulation to retain water, and the water and film forming polymer enable the urea to be applied to human skin such that the urea sits on the skin as a stable film and is gradually absorbed into the skin to alleviate dryness or cracking.

## Description

### FIELD OF INVENTION

This invention relates to urea based skin treatment. A preferred form of this invention relates to a urea based treatment for human skin, particularly for one's feet, and especially, but not necessarily, the heels.

### BACKGROUND

Many people suffer from dry skin at their feet. In some cases the skin may flake off or crack. It is known to apply urea based heel balms to the feet as it is thought this helps the skin to retain a healthy amount of water. Some known products rely on heavy oily emulsions to get the urea into the skin, but a downside is that they can be messy and may cause staining of bed linen, etc. They can also make the feet undesirably slippery, which may present a safety risk. It is an object of a preferred embodiment of the invention to go at least some way towards addressing at least some of these shortcomings. While this object applies to the preferred embodiment, it should be understood that the object of the invention per se is simply to provide the public with a useful choice. Therefore any objects applicable to the preferred embodiment should not be taken as a limitation on the scope of the claims to protection set out later in this document.

The term "comprises" or derivatives of this should not be interpreted as limiting. For example if used in relation to a combination of features it should be taken to indicate that optionally, but not necessarily, there may be additional features that have not been mentioned.

### SUMMARY OF INVENTION

According to one aspect of the invention there is provided a topical formulation for treating human skin, having:
- urea;
- film forming polymer;
- humectant;
- preservative; and
- water
the above ingredients being combined such that the preservative protects the formulation against bacterial invasion, the humectant assists the formulation to retain water, and the water and film forming polymer enable the urea to be applied to human skin such that the urea sits on the skin as a stable film and is gradually absorbed into the skin to alleviate dryness or cracking.

Optionally the formulation has 25% - 35% urea, by weight.

Optionally the formulation has about 30% urea, by weight.

Optionally the film forming polymer comprises a urethane polymer.

Optionally the film forming polymer comprises *Avalure 450* polymer.

Optionally the humectant comprises glycerine.

Optionally the preservative comprises one or more of phenoxyethanol, methyl paraben, ethyl paraben, propyl paraben and butyl paraben, and preferably a combination of all of these.

Optionally the preservative comprises *Salichem* preservative or *Phenonip* preservative.

According to a broader aspect of the invention, there is provided topical formulation for treating human skin, having:
- urea;
- film forming polymer; and
- water
the above ingredients being combined such that the water and film forming polymer enable the urea to be applied to human skin such that the urea sits on the skin as a stable film and is gradually absorbed into the skin to alleviate dryness or cracking.

### DETAILED DESCRIPTION

According to a preferred embodiment of the invention a topical formulation for heel skin is prepared from a mixture of the following ingredients:

| **Ingredient** | **Amount (grams)** | **Function** |
|---|---|---|
| Urea | 300 | therapeutic agent |
| water | 603 | Solvent |
| Avalure 450 polymer | 45 | Film forming polymer |
| Glycerine | 50 | humectant |
| *Salichem* preservative or *Phenonip* preservative | 2 | preservative |

It had not previously been known to formulate urea with a film forming polymer and, when done, the inventor found that it gave surprisingly good results.

The formulation is preferably prepared according to the following procedure -
a) obtain 603 gm of water at room temperature;
b) slowly add 300 gm of urea to the water, with constant stirring, until completely dissolved;
c) slowly add 45 gm of *Avalure 450* film forming polymer to the mix, with constant stirring, until a milky white homogeneous solution is achieved;
d) add 50 gm of glycerine to the mix, with constant stirring, until dissolved;
e) add 2 gm of a preservative to the mix (eg phenoxyethanol, methyl paraben, ethyl paraben, propyl paraben and butyl paraben), with constant stirring, until dissolved; and
f) continue mixing until a milky-white solution free of particles is achieved.

When created, the formulation is substantially waterproof (ie will not readily wash off the skin), is non-staining and does not have the sort of greasy or oily consistency of many prior art formulations.

As indicated in the table, the urea is preferably at an amount of 30% by weight. However in other embodiments of the invention there may be more, or less. For example the urea may be at 25-35% by weight.

As well as providing humectant functionality, the glycerine assists in making the formulation smooth. It imparts lubricant type characteristics.

It will be appreciated that alternative film forming polymers, humectants and preservatives may be used in place of those specifically mentioned above. However in each case the film forming polymer will be sufficient to give a stable urea containing film when applied to a human topically, and at least much of the urea able to be readily absorbed into the skin. Preferably the formulation is a spray-on one and is delivered by way of a spray dispenser.

While some preferred forms of the invention have been described by way of example, it should be understood that modifications and improvements can occur without departing from the scope of the following claims.

## Claims

1. A topical formulation for treating human skin, having:
• urea;
• film forming polymer;
• humectant;
• preservative; and
• water
the above ingredients being combined such that the preservative protects the formulation against bacterial invasion, the humectant assists the formulation to retain water, and the water and film forming polymer enable the urea to be applied to human skin such that the urea sits on the skin as a stable film and is gradually absorbed into the skin to alleviate dryness or cracking.

2. A formulation according to claim 1, having 25% - 35% urea, by weight.

3. A formulation according to claim 1 or 2, having about 30% urea, by weight.

4. A formulation according to claim 1, 2 or 3, having 30% urea, by weight.

5. A formulation according to any one of the preceding claims, wherein the film forming polymer comprises a urethane polymer.

6. A formulation according to claim 5, wherein the film forming polymer comprises *Avalure 450* polymer.

7. A formulation according to any one of the preceding claims, wherein the humectant comprises glycerine.

8. A formulation according to any one of the preceding claims, wherein the preservative comprises one or more of phenoxyethanol, methyl paraben, ethyl paraben, propyl paraben and butyl paraben, and preferably a combination of all of these.

9. A formulation according to any one of claims 1 to 7, wherein the preservative comprises *Salichem* preservative or *Phenonip* preservative.

10. A formulation according to claim 1, wherein the urea is in an amount of 25-35% by weight, the film forming polymer comprises a polyurethane substance and the humectant comprises glycerine.

11. A formulation according to claim 10, wherein the urea is in an amount of approximately 30% by weight.

12. A formulation substantially comprising ingredients in the following amounts: 300 grams Urea; 603 grams water; 45 grams Avalure 450 polymer; 50 grams Glycerine; and 2 grams Salichem or Phenonip preservative.

13. A topical formulation for treating human skin, having:
• urea;
• film forming polymer; and
• water
the above ingredients being combined such that the water and film forming polymer enable the urea to be applied to human skin such that the urea sits on the skin as a stable film and is gradually absorbed into the skin to alleviate dryness or cracking.
